# EUROPEAN PATENT APPLICATION

(11) **EP 4 005 752 A1**
(43) Date of publication of application: **01.06.2022**
(21) Application number: 20943360.6
(22) Date of filing: 14.08.2020
(51) Int. Cl.: B25J 11/00, B25J 9/16, A61L 2/24, A61L 2/18, A61L 2/10

(54) **MOVABLE ANTIVIRAL ROBOT AND METHOD FOR CONTROLLING SAME**

(30) Priority: 01.07.2020 KR 20200081132
(71) Applicant: Vision Semicon Co. Ltd., Daejeon 34026 (KR)
(72) Inventor: YUN, Sujung, Sejong 30064 (KR)
(74) Representative: RGTH
(86) International application number: PCT/KR2020/010862
(87) International publication number: WO 2022/004940

(57) **Abstract**

There is provided a mobile antiviral robot including a first antiviral unit to purify air, a second antiviral unit provided to spray a disinfectant solution, a third antiviral unit provided to emit ultraviolet radiation, a sensor unit to measure a pollution degree of the air, detect an object and generate position information, a learning unit to generate a path model by learning the position information, and a control unit to control a rotation speed of the blower fan, set a movement path according to a status of the object, set as a region of interest when the pollution degree is outside of a pollution threshold range, and generate a movement path from the path model.

## Description

### [Technical Field]

The present disclosure relates to a mobile antiviral robot and a method for controlling the same, and more particularly, to a mobile antiviral robot including a moving body which can move to different locations to purify viruses, and a method for controlling the same.

### [Background Art]

Todays, with the development of industries, environmental pollution is increasing, and there is a growing trend towards concentration of transportation and factories according to regional features, so air pollution is increasing day by day. Air pollution in homes and public buildings causes respiratory diseases and cancers. Additionally, air pollution causes severe skin diseases and skin cancers to children, and there is a tendency of increasing pathogen virulence in polluted air.

In relation to this, electric dust collector-type or filter-type air cleaners are being used to clean the polluted air, but common air cleaners are designed to work at specific fixed locations. In this case, air cleaners used in wide spaces drastically reduce in efficiency, and to clean the polluted air in the wide spaces, it is necessary to install air cleaners at multiple locations. In this case, there is inconvenience of having to continuously manage the plurality of air cleaners, and in terms of cost, purchasing and maintaining the plurality of air cleaners requires a high cost.

Accordingly, there is a need for approach to efficiently purify viruses in a wide space.

### [Disclosure]

### [Technical Problem]

The present disclosure is directed to providing a mobile antiviral robot including a moving body which can move to different locations to purify viruses, and a method for controlling the same.

### [Technical Solution]

An aspect of the present disclosure provides a mobile antiviral robot including a moving body to allow the mobile antiviral robot to move, and the mobile antiviral robot may include a first antiviral unit including a blower fan to induce movement of air and a purification filter to purify the air moving by the blower fan, a second antiviral unit provided to spray a disinfectant solution to disinfect an article, a third antiviral unit provided on a lower surface of the moving body to emit ultraviolet radiation towards a floor, a sensor unit to measure a pollution degree of the air, detect an object located in a movement direction of the moving body, and measure a position of the moving body to generate position information, a learning unit to generate a path model to present a movable area for the moving body by learning the position information changed with the movement of the moving body, and a control unit to control a rotation speed of the blower fan according to the pollution degree, when the object is detected, set a movement path according to a status of the detected object, when the pollution degree is outside of a preset pollution threshold range, set the position information as a region of interest, generate a movement path spaced a preset distance interval apart from the path model, and re-generate a straight line movement path spaced the predetermined distance apart from the previously generated movement path.

Additionally, the control unit may set the movement path to rotate in a same direction a preset number of times for a branch point found in the generated movement path, and when a number of rotations at the branch point reaches the preset number of times, may set the movement path to rotate the moving body in a different direction.

Additionally, the control unit may control the second antiviral unit to spray the disinfectant solution when the control unit receives a spray signal according to seat information of a location adjacent to the moving body from a seat control system provided to determine a seat status of a store indicating an empty seat and an occupied seat.

Additionally, when the object is detected, the control unit may control to stop the moving body, and may control to detect the object again after a preset time interval, and when the object is detected again, may control to rotate the moving body according to a preset steering angle.

Additionally, when it is determined that a movement distance of the moving body from a location at which a first object is detected and the moving body rotates according to a preset first steering angle to a location at which a second object is detected is within a preset distance range, the control unit may control to rotate the moving body according to a preset second steering angle.

Additionally, when the moving body arrives at a location more than the preset distance interval away from the region of interest, the control unit may set to move the moving body from the corresponding location to the region of interest along the previously generated movement path.

Additionally, when a number of arrivals at the location more than the preset distance interval away reaches a preset number of times, the control unit may increase the preset distance interval by a predetermined amount.

Additionally, the control unit may set any one of branch points along the generated movement path as a target point, and may set to move to the region of interest when arriving at the branch point set as the target point, and the branch point present in the movement path may be set as the target point in a sequential order in an ascending order of distance from the region of interest.

Another aspect of the present disclosure provides a method for controlling a mobile antiviral robot including a moving body to allow the mobile antiviral robot to move, and the method may include purifying air using a purification filter, wherein the air moves by a blower fan which rotates to induce the movement of the air, spraying a disinfectant solution to disinfect an article, emitting ultraviolet radiation from a lower surface of the moving body towards a floor, measuring a pollution degree of the air, measuring a position of the moving body to generate position information, detecting an object located in a movement direction of the moving body, controlling a rotation speed of the blower fan according to the pollution degree, generating a path model to present a movable area for the moving body by learning the position information changed with the movement of the moving body, setting a movement path according to a status of the detected object when the object is detected, setting the position information as a region of interest when the pollution degree is outside of a preset pollution threshold range, generating a movement path spaced a preset distance interval apart from the path model, and re-generating a straight line movement path spaced the predetermined distance apart from the previously generated movement path.

Additionally, generating the movement path in a grid shape may include setting any one of branch points along the generated movement path as a target point, and setting to move to the region of interest when arriving at the branch point set as the target point, and the branch point present in the movement path may be set as the target point in a sequential order in an ascending order of distance from the region of interest.

Additionally, spraying the disinfectant solution to disinfect the article may be performed when receiving a spray signal according to seat information of a location adjacent to the moving body from a seat control system provided to determine a seat status of a store indicating an empty seat and an occupied seat.

Additionally, generating the movement path in a grid shape may include setting the movement path to rotate in a same direction a preset number of times for the branch point found in the generated movement path, and when a number of rotations at the branch point reaches the preset number of times, setting the movement path to rotate the moving body in a different direction.

### [Advantageous Effects]

According to an aspect of the present disclosure, there is provided a mobile antiviral robot including a moving body which can move to different locations to purify viruses, and a method for controlling the same.

### [Description of Drawings]

FIGS. 1 to 3 are schematic diagrams of a mobile antiviral robot according to an embodiment of the present disclosure.
FIG. 4 is a control block diagram of a mobile antiviral robot according to an embodiment of the present disclosure.
FIG. 5 is a block diagram showing a process of controlling an antivirus unit by a control unit of FIG. 4.
FIG. 6 is a block diagram showing a process of controlling to move a moving body by a control unit of FIG. 4.
FIG. 7 is a block diagram showing a process of generating a path model by a control unit of FIG. 4.
FIGS. 8 to 11 are schematic diagrams showing an embodiment of an antivirus unit of FIG. 4.
FIGS. 12 and 13 are schematic diagrams showing an embodiment of a movement path generated by a control unit of FIG. 4.
FIG. 14 is a flowchart of a method for controlling a mobile antiviral robot according to an embodiment of the present disclosure.

### [Best Mode]

The following detailed description of the present disclosure is made with reference to the accompanying drawings, in which particular embodiments for practicing the present disclosure are shown for illustration purposes. These embodiments are described in sufficiently detail for those skilled in the art to practice the present disclosure. It should be understood that various embodiments of the present disclosure are different but do not need to be mutually exclusive. For example, particular shapes, structures and features described herein in connection with one embodiment may be implemented in other embodiment without departing from the spirit and scope of the present disclosure. It should be further understood that changes may be made to the positions or placement of individual elements in each disclosed embodiment without departing from the spirit and scope of the present disclosure. Accordingly, the following detailed description is not intended to be taken in limiting senses, and the scope of the present disclosure, if appropriately described, is only defined by the appended claims along with the full scope of equivalents to which such claims are entitled. In the drawings, similar reference signs denote same or similar functions in many aspects.

Hereinafter, preferred embodiments of the present disclosure will be described in more detail with reference to the accompanying drawings.

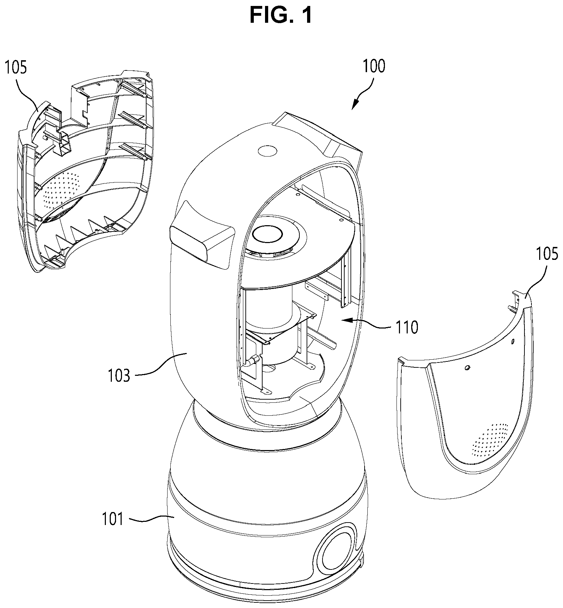

FIGS. 1 to 3 are schematic diagrams of a mobile antiviral robot according to an embodiment of the present disclosure.

The mobile antiviral robot 100 may include a moving body 101, and accordingly, the mobile antiviral robot 100 may move by the moving body 101, and purify air in a space in which the mobile antiviral robot 100 is located.

Here, the moving body 101 may be provided to move by the control of the mobile antiviral robot 100, and in this instance, the moving body 101 may be provided such that its direction can be changed by the steering control of the mobile antiviral robot 100.

The mobile antiviral robot 100 may include a rotatable blower fan to allow air to enter an internal space formed by at least one cover 105, and the mobile antiviral robot 100 may include a purification filter to purify air entering the internal space by the blower fan.

To this end, the moving body 101 may further include a body 103 to which the cover 105 is secured, and the body 103 may be provided such that it is formed integrally with the moving body 101 or separately from the moving body 101. Additionally, the moving body 101 may further include a power supplier and may work using the power supplied from the power supplier.

Here, the cover 105 may be provided such that it can be assembled in the body 103 of the moving body 101, and the cover 105 may include an air hole to allow air to enter the internal space formed by the cover 105.

Meanwhile, the internal space may be provided separately from the cover 105, and for example, the internal space may be provided using a cylindrical air passage.

As described above, the internal space may be implemented in other embodiment without departing from the spirit and scope of the present disclosure in relation to an embodiment of the particular shape, structure and feature as described herein.

In relation to this, the blower fan may be provided on the upper side of the internal space formed by the cover 105, and may rotate to force air entering the internal space out of the internal space.

In this case, the mobile antiviral robot 100 may control to allow air to enter the lower side or the side of the internal space by the blower fan, and the mobile antiviral robot 100 may control to allow the incoming air to pass through the blower fan and exit through the upper side of the internal space.

Additionally, the blower fan may be provided on the lower side of the internal space formed by the cover 105, and may rotate to move air inside the internal space to the upper side of the internal space. In this instance, the internal space may further include an air inlet to allow air to enter from the lower side or the side of the internal space.

In this case, the mobile antiviral robot 100 may control the blower fan to move air inside the internal space to the upper side of the internal space by the blower fan, and force the air having moved to the upper side of the internal space out of the internal space, and accordingly, the mobile antiviral robot 100 may let air into the internal space from the lower side or the side of the internal space.

In this instance, the blower fan may be provided such that its rotation speed is controlled by the mobile antiviral robot 100.

Meanwhile, the purification filter may be installed in the internal space formed by the cover 105. In this instance, the purification filter may be provided to capture or filter out pollutants in air.

In relation to this, the purification filter may include a plurality of single filters in different shapes, and here, the plurality of single filters may be provided as filters for capturing or filtering out different pollutants.

For example, the single filter may include a pre filter for capturing dust with large particles in air, an activated carbon filter for capturing odor, hazardous gas or particles of Total Volatile Organic Compounds (TVOC) in air, and a H13 High Efficiency Particulate Air Filter (HEPA Filter) for capturing particles in air, for example, ultrafine dust of 2.5 micrometers or less, and in this case, the purification filter may be provided in the form of a triple filter including the pre filter, the activated carbon filter and the H13 HEPA filter.

As described above, the single filter may be of a mesh type to allow air to pass through and filter out pollutants, and in this instance, the single filter may be made of metal or synthetic resin.

Additionally, the purification filter may include the plurality of different single filters provided for different purposes according to the feature of the space in which the mobile antiviral robot 100 works, and it is obvious that the purification filter may include a plurality of different well-known filters.

Meanwhile, the purification filter may be provided to be secured to the inner side of the cover 105 that forms the internal space, and additionally, the purification filter may be secured around the internal space formed by the cover 105. Additionally, the purification filter may be provided in the internal space so that air entering from the lower side or the side of the internal space by the operation of the blower fan passes through the purification filter.

In this case, the internal space formed by the cover 105 may be provided with an assembly in the shape of a tube with a hollow center to which the purification filter is secured, and accordingly, the mobile antiviral robot 100 may control the blower fan to move air purified by the purification filter into the assembly in the shape of a tube with a hollow center, and force the purified air inside the assembly out of the internal space formed by the cover 105.

In this instance, the purification filter may be replaceably provided.

Meanwhile, the mobile antiviral robot 100 may include a disinfection lamp provided on the lower surface of the moving body 101 to emit UV radiation towards the floor on which the moving body 101 moves.

In this case, the mobile antiviral robot 100 may disinfect the floor using the disinfection lamp when the moving body 101 stands by or while the moving body 101 is moving.

Here, the disinfection lamp may be provided to emit UV radiation in the wavelength range of 100 nanometers to 280 nanometers, and for example, the disinfection lamp may be a UV-C disinfection lamp.

The mobile antiviral robot 100 may further include a sprayer to spray a disinfectant solution onto an article such as a table and a chair.

In this instance, the sprayer may be provided outside of the internal space, and the cover 105 may include a hole through which the disinfectant solution sprayed from the sprayer is delivered out of the mobile antiviral robot 100. In this instance, the sprayer may further include a nozzle to spray the disinfectant solution out of the mobile antiviral robot 100, and in this case, the cover 105 may include the hole in which the nozzle of the sprayer is positioned.

Additionally, when the mobile antiviral robot 100 includes the sprayer to spray the disinfectant solution onto the article such as the table and the chair, the mobile antiviral robot 100 may spray the disinfectant solution onto the article such as the table and chair determined to be empty.

When the mobile antiviral robot 100 is positioned in a store, for example, a cafe, this may be understood as spraying the disinfectant solution onto the table and chair determined to be empty, and in this instance, the mobile antiviral robot 100 may spray the disinfectant solution in response to a spray signal of the disinfectant solution received from a seat control system connected with a wireless or wired network.

In this instance, the seat control system may be a system provided to determine the seat status in the store indicating an empty seat and an occupied seat, and the seat control system may be replaced with an information processing device connected to the mobile antiviral robot 100, for example, a server, a computer and a mobile terminal.

In relation to this, the spray signal may be a signal indicating that a seat adjacent to the mobile antiviral robot 100 is an empty seat, and to this end, the mobile antiviral robot 100 may receive input of seat information indicating the seat position in advance.

Accordingly, the mobile antiviral robot 100 may transmit identification information for the seat adjacent to the mobile antiviral robot 100 to the seat control system, and in this instance, when the mobile antiviral robot 100 receives the spray signal for the corresponding seat from the seat control system, the mobile antiviral robot 100 may spray the disinfectant solution.

Additionally, the mobile antiviral robot 100 may be provided to recognize a marker provided in at least one article of the ceiling above the seat, the table or the chair in the store. In this instance, the mobile antiviral robot 100 may include a 2-dimensional (2D) camera to recognize the marker on one side of the body 103.

For example, when the marker is provided in the ceiling of the store, the mobile antiviral robot 100 may include the 2D camera on the body 103 of the mobile antiviral robot 100 to recognize the marker.

Here, the marker provided in at least one article of the ceiling, the table or the chair may be provided to transmit the identification information for the seat to the mobile antiviral robot 100, and accordingly, the mobile antiviral robot 100 may transmit the identification information for the seat generated by recognizing the marker to the seat control system, and in this instance, when the mobile antiviral robot 100 receives the spray signal for the corresponding seat from the seat control system, the mobile antiviral robot 100 may spray the disinfectant solution.

In relation to this, the seat control system may include a status change switch in each seat in the store, and in this instance, the seat control system may change the status of the seat according to a status change signal generated when a user presses the status change switch.

For example, the seat control system may set the initial status for the seat to an empty seat status, and when the status change signal is generated in response to the user input, the seat control system may switch between the empty seat status and an occupied seat status.

In this instance, the status change signal may include the identification information to identify the seat having the status change switch, and accordingly, the seat control system may control the seat status of any one of a plurality of seats in the store.

Meanwhile, the seat control system may identify customers using a photographing device equipped in the store, and in this case, may determine an occupied seat and an empty seat by learning the customers' behavior patterns.

The sprayer may be provided to spray the disinfectant solution in response to the external input spray signal, and here, the spray signal may be a signal using a wireless network such as Wireless Fidelity (Wi-Fi) and Bluetooth, and the spray signal may be generated by a device which can connect to the wireless network, for example, a computer, a smartphone and a tablet.

In this instance, the mobile antiviral robot 100 may be connected to the device which generates the spray signal with the wireless network.

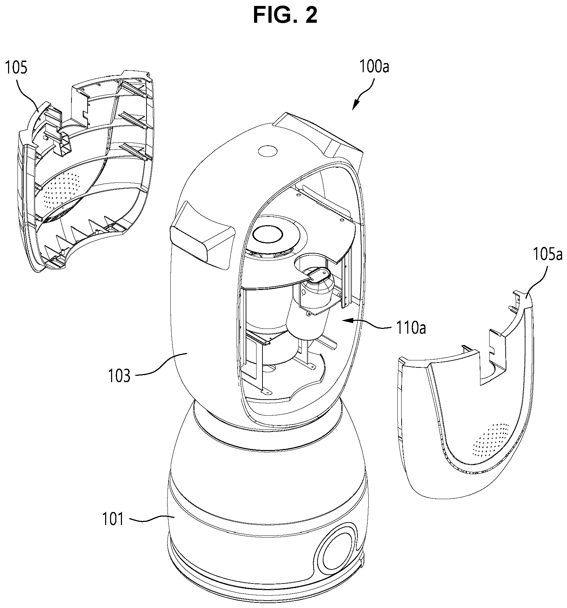

In relation to this, referring to FIG. 1, the mobile antiviral robot 100 may be understood as including the sprayer on the front side of the antiviral unit 110, and referring to FIG. 2, the mobile antiviral robot 100a may include the sprayer on two sides of the antiviral unit 110a, and accordingly, FIG. 2 may be understood as another embodiment of the mobile antiviral robot 100.

As described above, the sprayer of the mobile antiviral robot 100 may be implemented in other embodiment without departing from the spirit and scope of the present disclosure in relation to an embodiment of the particular shape, structure and feature as described herein.

The mobile antiviral robot 100 may include a pollution degree measurement sensor to measure the pollution degree of air entering the internal space by the rotation of the blower fan.

To this end, the mobile antiviral robot 100 may include a sensor which emits light using an optical sensor or a laser light source, outputs light scattered by fine or ultrafine dust in air as an electrical signal through a photodiode, and determines the pollution degree of air according to the size of the output electrical signal.

Additionally, the mobile antiviral robot 100 may measure the pollution degree by using a sensor which detects the concentration of pollutants in air, for example, hydrogen sulfide, ammonia, dioxide carbon and methane, and the mobile antiviral robot 100 may measure the pollution degree for each pollutant by using a plurality of sensors to measure the concentration of different materials.

In this instance, the pollution degree measurement sensor may be provided to measure the pollution degree from air before pollutants in air is captured by the purification filter.

Meanwhile, the mobile antiviral robot 100 may include an object detection sensor to detect an object by transmitting a detection signal and receiving the detection signal reflected by the object.

Here, the object may refer to an obstacle which blocks the movement of the moving body 101, for example, a person, a wall and a table.

To this end, the mobile antiviral robot 100 may use a sensor which measures a time interval from the transmission time of the ultrasonic, infrared, laser, radio detection and ranging (radar) detection signal to the reception time of the detection signal reflected by an object, and accordingly, the mobile antiviral robot 100 may determine the distance to the object according to the measured time interval.

Additionally, the mobile antiviral robot 100 may detect the object by detecting a bumper disposed around the moving body 101 and the pressure generated from the bumper.

Additionally, the mobile antiviral robot 100 may include the object detection sensor installed at any location of the body, for example, the front side, the lower side or the upper side of the body, and in this instance, the mobile antiviral robot 100 may include a plurality of object detection sensors at different locations.

In this instance, the object detection sensor may be provided to transmit the detection signal towards the movement direction of the moving body 101, and accordingly, the mobile antiviral robot 100 may detect an object located in front of the mobile antiviral robot 100.

Meanwhile, the mobile antiviral robot 100 may include a position measurement sensor to measure the position of the moving body to generate position information, and in this instance, the mobile antiviral robot 100 may generate the position information according to a preset time cycle.

Additionally, the mobile antiviral robot 100 may generate the position information according to a preset distance cycle, and in this case, the mobile antiviral robot 100 may control to generate the position information according to the predetermined distance cycle by applying different time cycles depending on the movement speed of the moving body 101 to determine the preset distance cycle.

In this instance, the mobile antiviral robot 100 may measure its position using Global Position (GPS) and Global Navigation Satellite System (GNSS).

Here, GNSS is a technique in which a receiver receives a satellite signal from a satellite and the position of the receiver is determined by calculating the distance to the satellite from the received satellite signal, and in this instance, GNSS uses a method of calibrating the position of the receiver using a distance difference between a regular observation station and the receiver and the position information of the regular observation station.

Additionally, the mobile antiviral robot 100 may receive each signal from a plurality of signal transmitters installed at different locations, and calculate the distance to the signal transmitter having transmitted each signal according to the intensity of the received signal, and in this case, the mobile antiviral robot 100 may be provided to calculate its position according to a difference between the calculated distances by the plurality of signal transmitters.

In this instance, the signal transmitter may be a device which transmits a wireless network signal, for example, Wi-Fi.

The mobile antiviral robot 100 may control the rotation speed of the blower fan according to the pollution degree measured by the pollution degree measurement sensor.

To this end, the mobile antiviral robot 100 may determine the pollution level according to the pollution degree measured by the pollution degree measurement sensor, and the mobile antiviral robot 100 may control the rotation speed of the blower fan according to the determined pollution level.

Here, the pollution level may be set such that the pollution degree is classified into at least one level according to the numerical range indicated by the pollution degree.

In this instance, when different styles of pollution degree measurement sensors are provided, each may be provided to determine the pollution level according to the pollution degree measured by each pollution degree measurement sensor, and the mobile antiviral robot 100 may control the rotation speed of the blower fan based on the plurality of pollution levels determined according to the pollution degree measured by different styles of pollution degree measurement sensors.

For example, the mobile antiviral robot 100 may control the rotation speed of the blower fan according to the pollution level determined to be the highest one of the plurality of pollution levels, and additionally, the mobile antiviral robot 100 may calculate an average pollution level from the plurality of pollution levels, and control the rotation speed of the blower fan according to the calculated average pollution level.

Additionally, the mobile antiviral robot 100 may set a weight for each pollution for each of the plurality of pollution degrees measured by different methods, and in this case, the mobile antiviral robot 100 determine the pollution level for each pollution degree by applying the weight to each of the plurality of pollution degrees measured by different methods, and accordingly, the mobile antiviral robot 100 may control the rotation speed of the blower fan according to the pollution level indicating the highest one of the plurality of pollution levels.

When the object is detected by the object detection sensor, the mobile antiviral robot 100 may set a movement path according to the status of the detected object, and the mobile antiviral robot 100 may control to move the moving body along the set movement path.

Here, when the distance interval with the object measured from the object detection sensor is shorter than a preset threshold, the mobile antiviral robot 100 may determine that the object is detected, and when the time interval from the transmission time of the detection signal from the object detection sensor to the reception time of the detection signal reflected by the object is shorter than the preset threshold, it may be determined that the object is detected.

Additionally, when the distance interval with the object measured from the object detection sensor is longer than the preset threshold or the distance to the object is not measured, the mobile antiviral robot 100 may determine that the object is not detected.

Accordingly, when the object is detected, the mobile antiviral robot 100 may control to stop the moving body 101, and the mobile antiviral robot 100 may control to carry out object detection again after a preset time interval.

In this instance, when the object is detected again after controlling to stop the moving body 101 due to the presence of the detected object, the mobile antiviral robot 100 may control to rotate the moving body 101 according to a preset steering angle.

In relation to this, when the mobile antiviral robot 100 controls to rotate the moving body 101 according to the preset steering angle, the mobile antiviral robot 100 may control to rotate the moving body 101 in the same direction as the previous rotation direction.

Accordingly, the mobile antiviral robot 100 may carry out object detection towards the changed direction by the rotation of the moving body 101, and when the object is not detected from the changed direction, the mobile antiviral robot 100 may control to move the moving body 101 towards the changed direction.

For example, when the object is detected, the mobile antiviral robot 100 may control to stop the moving body 101, and when the object is detected again by the object detection carried out again after the predetermined time interval from the stop of the moving body 101, may control to rotate the moving body 101 left or right by 60°.

Meanwhile, when the object is detected by the object detection towards the changed direction by the rotation of the moving body 101, the mobile antiviral robot 100 may control to carry out object detection again after the preset time interval, and when the object is detected again, the mobile antiviral robot 100 may control to rotate the moving body 101 according to the preset steering angle.

In relation to this, when it is determined that the movement distance of the moving body 101 from the location at which the first object is detected from the object detection sensor and the moving body 101 rotates according the preset first steering angle to the location at which the second object is detected is within the preset distance range, the mobile antiviral robot 100 may control to rotate the moving body 101 according to the preset second steering angle.

For example, when the first object is detected from the object detection sensor, the mobile antiviral robot 100 may control to rotate the moving body 101 by 60° according to a series of control processes, and when the movement distance of the moving body 101 from the location at which the moving body rotates 60° to the location at which the second object is detected is within the preset distance range, the mobile antiviral robot 100 may control to rotate the moving body 101 by 30° according to a series of control processes.

When the pollution degree measured from the pollution degree measurement sensor is outside of a preset pollution threshold range, the mobile antiviral robot 100 may set the current position information measured from the position measurement sensor as a region of interest, and the mobile antiviral robot 100 may reset the movement path according to the region of interest.

In relation to this, when the mobile antiviral robot 100 controls to rotate the moving body 101 a preset number of times, the mobile antiviral robot 100 may control to move the moving body 101 to the set region of interest.

To this end, the mobile antiviral robot 100 may control to rotate the moving body 101 towards the region of interest by comparing the position information measured from the position measurement sensor with the position information set as the region of interest.

In this instance, when an object is detected from the object detection sensor while the moving body 101 is moving to the region of interest, the mobile antiviral robot 100 may control to stop the moving body 101, and the mobile antiviral robot 100 may control to carry out object detection again after the preset time interval.

In this instance, when the object is detected again after controlling to stop the moving body 101 due to the presence of the detected object, the mobile antiviral robot 100 may control to rotate the moving body 101 according to the preset steering angle, and the mobile antiviral robot 100 may move a preset threshold distance interval according to the rotation direction of the moving body 101.

Accordingly, the mobile antiviral robot 100 may control to rotate the moving body 101 towards the region of interest by comparing the position information measured at the location to which the moving body 101 has moved the preset threshold distance interval with the position information set as the region of interest.

Meanwhile, when arriving at the location set as the region of interest, the mobile antiviral robot 100 may control to rotate the moving body 101 according to the preset steering angle.

The mobile antiviral robot 100 may generate a path model to present a movable area for the moving body 101 by learning the position information changed with the movement of the moving body 101 along the movement path.

To this end, the mobile antiviral robot 100 may use an unsupervised learning technique, for example, Principal Components Analysis (PCA) which analyzes input information and generates a pattern of the input information.

Here, PCA is a technique which calculates a line or surface showing the best representation of a plurality of information located in a dimensional space.

Accordingly, the mobile antiviral robot 100 may control to move the moving body 101 along the movement path set based on the generated path model.

In this instance, the mobile antiviral robot 100 may generate the path model according to a plurality of position information indicating the outermost side among the position information generated by the movement of the moving body 101.

In this case, the path model may be generated to present the movable path along an outer wall of the space in which the mobile antiviral robot 100 is installed. Here, the outer wall may refer to an obstacle such as a wall located at the outermost side among locations to which the moving body 101 can move in the space in which the mobile antiviral robot 100 is installed.

Meanwhile, the mobile antiviral robot 100 may generate the movement path spaced a preset distance interval apart from the path model generated along the outer side of the collected position information.

Here, the preset distance interval may be set to represent an area which air is purified by the mobile antiviral robot 100, and accordingly, the mobile antiviral robot 100 may generate the inner movement path spaced the predetermined distance apart from the outer side of the collected position information.

In this instance, the mobile antiviral robot 100 may generate the straight line movement path spaced the predetermined distance apart from the previously generated movement path. In this case, the mobile antiviral robot 100 may generate the movement path in a grid shape within the movement path generated according to the position information of the outer side.

For example, the mobile antiviral robot 100 may generate a second movement path representing a straight line path spaced the predetermined distance apart from a first movement path based on the first movement path generated to represent the outer side, and the mobile antiviral robot 100 may generate a third movement path representing a straight line path spaced the predetermined distance apart from the second movement path based on the second movement path.

Additionally, when the location spaced the predetermined distance apart from the outer side of the collected position information is determined to be an area impossible to move, the mobile antiviral robot 100 may generate the movement path to move the moving body 101 to the closest outer movement path.

Accordingly, the mobile antiviral robot 100 may set the movement path to move the moving body 101 along the movement path generated to represent the outer side of the collected position information and the movement path generated within the movement path according to the position information of the outer side in an alternating manner.

Meanwhile, the mobile antiviral robot 100 may set, as a branch point, a point of intersection of different lines indicating the movable path of the moving body in the movement path of a grid shape.

Additionally, the mobile antiviral robot 100 may set the movement path to rotate and move in the same direction at the branch point found in the generated movement path, and in this instance, when the previous movement path is found, the mobile antiviral robot 100 may set the movement path to rotate and move in a different direction.

Additionally, when the movement path of each direction found from the branch point is determined to be the previous movement path, the mobile antiviral robot 100 may set the movement path to move along the immediately previous movement path, and when arriving at the branch point where there is a movement path that has never been moved before, may set the movement path to rotate and move toward the corresponding movement path.

To this end, the mobile antiviral robot 100 may store the position information generated in a time series, and determine the previous movement path by comparing the position information generated for a period of time with the movement path.

Additionally, the mobile antiviral robot 100 may set the movement path to rotate in the same direction the preset number of times for the branch point found in the generated movement path, and in this case, when the number of rotations at the branch point reaches the preset number of times, the mobile antiviral robot 100 may set the movement path to rotate in a different direction.

Meanwhile, when the region of interest is set, the mobile antiviral robot 100 may use the movable path according to the path model to move from another region to the region of interest.

In relation to this, when arriving at a location more than the preset distance interval away from the region of interest, the mobile antiviral robot 100 may set to move from the corresponding location to the region of interest along the previously generated movement path.

In this case, when the number of arrivals at the location more than the preset distance interval away from the region of interest reaches a preset number of times, the mobile antiviral robot 100 may increase the preset distance interval by a predetermined amount.

Here, the region of interest may be set as a point on the generated movement path, and accordingly, the mobile antiviral robot 100 may generate a circle having the preset distance interval from the region of interest as a radius, and when the measured position information is beyond the generated circle, may be set to move to the region of interest.

In this instance, when the number of times it is beyond the circle according to the preset distance interval reaches the preset number of times, the mobile antiviral robot 100 may increase the preset distance interval by the predetermined amount.

Meanwhile, the mobile antiviral robot 100 may move along other movement path than the movement path that has been used before among at least one possible movement path to the region of interest.

To this end, the mobile antiviral robot 100 may recognize directions of different possible movement paths from the region of interest, and accordingly, the mobile antiviral robot 100 may select the directions of the movement paths recognized from the region of interest in a sequential order, and move in different directions. This may be understood as selecting a movable direction in a sequential order each time the mobile antiviral robot 100 arrives at any one branch point.

Additionally, the mobile antiviral robot 100 may recognize the branch point found along the movement path set from the region of interest, and accordingly, the mobile antiviral robot 100 may select the direction of the movement path including the branch point recognized along the movement path set from the region of interest in the process of selecting the direction of the movement path recognized from the region of interest in a sequential order.

Here, the mobile antiviral robot 100 may recognize the n-th branch point among the branch points found in a sequential order along the movement path set from the region of interest, and for example, the mobile antiviral robot 100 may set the branch point recognized for the first time among the branch points found along the movement path set from the region of interest as one of the directions of the movement path recognized from the region of interest.

Additionally, when the region of interest is set to move from a location to the region of interest, the mobile antiviral robot 100 may select the direction of the movement path with the smallest number of rotations and be set to move to the region of interest.

Additionally, the mobile antiviral robot 100 may set, as a target point, any one of the branch points according to the generated movement path, and when arriving at the branch point set as the target point, the mobile antiviral robot 100 may be set to move to the region of interest.

In this instance, the mobile antiviral robot 100 may set the branch point included in the previously set movement path as a passage point, and in this case, the mobile antiviral robot 100 may set, as the target point, any one branch point except the branch point set as the passage point.

Additionally, the mobile antiviral robot 100 may set, as the target point, the branch point present in the generated movement path in a sequential order in an ascending order of the distance from the region of interest, and the mobile antiviral robot 100 may set, as the target point, the branch point present in the generated movement path in a sequential order in a descending order of the distance from the region of interest.

Meanwhile, when a plurality of regions of interest is set, the mobile antiviral robot 100 may set the movement path for move from a region of interest to another region of interest.

In this instance, the mobile antiviral robot 100 may set the movement path to move from a region of interest to another region of interest along a different movement path from the previous movement path.

Additionally, the mobile antiviral robot 100 may select another region of interest selected to move from the certain region of interest as the region of interest in a preset time order, and the mobile antiviral robot 100 may select another region of interest from the certain region of interest to move in a sequential order in an ascending order of the pollution degree measured in the region of interest.

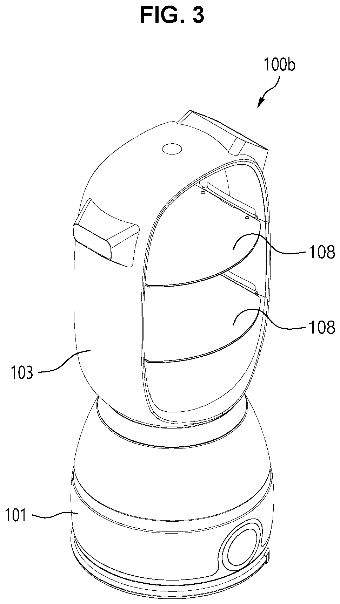

Meanwhile, referring to FIG. 3, the mobile antiviral robot 100b may remove the antiviral unit 110 provided within the body 103, and may assemble a shelf 108, and in this case, the mobile antiviral robot 100b may be used as a shelf that can move by itself.

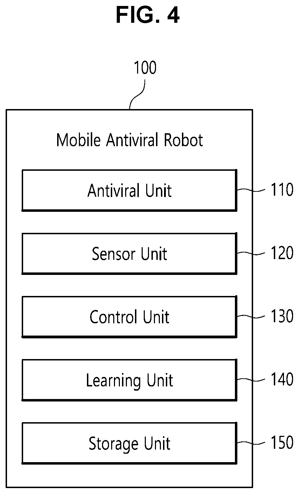

FIG. 4 is a control block diagram of the mobile antiviral robot according to an embodiment of the present disclosure.

The mobile antiviral robot 100 may include an antiviral unit 110, a sensor unit 120, a control unit 130, a learning unit 140 and a storage unit 150, and here, the antiviral unit 110 may include a first antiviral unit, a second antiviral unit and a third antiviral unit.

The first antiviral unit may include the rotatable blower fan to allow air to enter the internal space formed by the at least one cover 105, and the first antiviral unit may include the purification filter to purify air entering the internal space by the blower fan.

The blower fan may be provided on the upper side of the internal space formed by the cover 105, and may rotate to force air entering the internal space out of the internal space.

In this case, the first antiviral unit may be controlled to allow air to enter the lower side or the side of the internal space by the blower fan, and the first antiviral unit may be controlled to allow the incoming air to pass through the blower fan and exit through the upper side of the internal space.

Additionally, the blower fan may be provided on the lower side of the internal space formed by the cover 105, and may rotate to move air inside the internal space to the upper side of the internal space.

In this case, the first antiviral unit may be controlled to move air inside the internal space to the upper side of the internal space by the blower fan, and force the air having moved to the upper side of the internal space out of the internal space, and accordingly, the first antiviral unit may let air into the internal space from the lower side or the side of the internalspace.

In this instance, the blower fan may be provided such that its rotation speed is controlled by the control unit 130.

Meanwhile, the purification filter may be installed in the internal space formed by the cover 105. In this instance, the purification filter may be provided to capture or filter out pollutants in air.

In relation to this, the purification filter may include a plurality of single filters in different shapes, and here, the plurality of single filters may be provided as filters for capturing or filtering out different pollutants.

Meanwhile, the purification filter may be provided to be secured to the inner side of the cover 105 that forms the internal space, and additionally, the purification filter may be secured around the internal space formed by the cover 105. Additionally, the purification filter may be provided in the internal space so that air entering from the lower side or the side of the internal space passes through the purification filter by the operation of the blower fan.

In this case, the internal space formed by the cover 105 may be provided with an assembly in the shape of a tube with a hollow center to which the purification filter is secured, and accordingly, the first antiviral unit may control the blower fan to move air purified by the purification filter into the assembly in the shape of a tube with a hollow center, and force the purified air inside the assembly out of the internal space formed by the cover 105.

In this instance, the purification filter may be replaceably provided.

The second antiviral unit may be a sprayer which sprays the disinfectant solution onto the article such as the table and the chair.

In this instance, the sprayer may be provided outside of the internal space, and the cover 105 may include the hole through which the disinfectant solution sprayed from the sprayer 115 is delivered out of the mobile antiviral robot 100. In this instance, the sprayer may further include the nozzle to spray the disinfectant solution out of the mobile antiviral robot 100, and in this case, the cover 105 may include the hole in which the nozzle of the sprayer is positioned.

The third antiviral unit may be a disinfection lamp provided on the lower surface of the moving body 101 to emit UV radiation towards the floor on which the moving body 101 moves.

In this case, the third antiviral unit may disinfect the floor using the disinfection lamp when the moving body 101 stands by or while the moving body 101 is moving.

The sensor unit 120 may include the pollution degree measurement sensor to measure the pollution degree of air entering the internal space by the rotation of the blower fan.

In this instance, the pollution degree measurement sensor may be provided to measure the pollution degree from air before pollutants in the air are captured by the purification filter.

Meanwhile, the sensor unit 120 may include the object detection sensor to transmit a detection signal and receive the detection signal reflected by an object to detect the object.

In this instance, the object detection sensor may be provided to transmit the detection signal towards the movement direction of the moving body 101, and accordingly, the sensor unit 120 may detect an object located in front of the sensor unit 120.

Meanwhile, the sensor unit 120 may include the position measurement sensor to measure the position of the moving body to generate position information.

The sensor unit 120 may recognize a marker provided in at least one article of the ceiling above the seat, the table or the chair in the store. In this instance, the sensor unit 120 may include the 2D camera on one side of the body 103 to recognize the marker.

For example, when the marker is provided in the ceiling of the store, the sensor unit 120 may include the 2D camera on the body 103 of the mobile antiviral robot 100 to recognize the marker.

The control unit 130 may control the rotation speed of the blower fan according to the pollution degree measured by the pollution degree measurement sensor.

To this end, the control unit 130 may determine the pollution level according to the pollution degree measured by the pollution degree measurement sensor, and the control unit 130 may control the rotation speed of the blower fan according to the determined pollution level.

Here, the pollution level may be set such that the pollution degree is classified into at least one level according to the numerical range indicated by the pollution degree.

In this instance, when different styles of pollution degree measurement sensors are provided, each may be provided to determine the pollution level according to the pollution degree measured by each pollution degree measurement sensor, and the control unit 130 may control the rotation speed of the blower fan based on the plurality of pollution levels determined according to the pollution degree measured by different styles of pollution degree measurement sensors.

Additionally, when the antiviral unit 110 includes the sprayer to spray the disinfectant solution onto the article such as the table and the chair, the control unit 130 may spray the disinfectant solution onto the article such as the table and chair determined to be empty.

When the mobile antiviral robot 100 is positioned in the store, for example, a cafe, this may be understood as spraying the disinfectant solution onto the table and chair determined to be empty, and in this instance, the control unit 130 may control to spray the disinfectant solution in response to a spray signal of the disinfectant solution received from the seat control system connected with the wireless or wired network.

To this end, the control unit 130 may receive input of seat information indicating the seat position in advance.

Accordingly, the control unit 130 may transmit identification information for a seat adjacent to the mobile antiviral robot 100 to the seat control system, and in this instance, when the control unit 130 receives the spray signal for the corresponding seat from the seat control system, the control unit 130 may control to spray the disinfectant solution.

Additionally, in case that the marker is provided in the ceiling of the store, when the sensor unit 120 recognizes the marker, the control unit 130 may generate the identification information for the seat according to the corresponding marker, and the control unit 130 may transmit the extracted identification information to the seat control system, and in this instance, when the control unit 130 receives the spray signal for the corresponding seat from the seat control system, the control unit 130 may control to spray the disinfectant solution.

When the object is detected by the object detection sensor, the control unit 130 may set a movement path according to the status of the detected object, and the control unit 130 may control to move the moving body along the set movement path.

Accordingly, when the object is detected, the control unit 130 may control to stop the moving body 101, and the control unit 130 may control to carry out object detection again after the preset time interval.

In this instance, when the object is detected again after controlling to stop the moving body 101 due to the presence of the detected object, the control unit 130 may control to rotate the moving body 101 according to the preset steering angle.

In relation to this, when the control unit 130 controls to rotate the moving body 101 according to the preset steering angle, the control unit 130 may control to rotate the moving body 101 in the same direction as the previous rotation direction.

Accordingly, the control unit 130 may carry out object detection towards the changed direction by the rotation of the moving body 101, and when the object is not detected from the changed direction, the control unit 130 may control to move the moving body 101 towards the changed direction.

Meanwhile, when the object is detected by the object detection towards the changed direction by the rotation of the moving body 101, the control unit 130 may control to carry out object detection again after the preset time interval, and when the object is detected again, may control to rotate the moving body 101 according to the preset steering angle.

In relation to this, when it is determined that the movement distance of the moving body 101 from the location at which the first object is detected from the object detection sensor and the moving body 101 rotates according to the preset first steering angle to the location at which the second object is detected is within the preset distance range, the control unit 130 may control to rotate the moving body 101 according to the preset second steering angle.

When the pollution degree measured from the pollution degree measurement sensor is outside of the preset pollution threshold range, the control unit 130 may set the current position information measured from the position measurement sensor as a region of interest, and the control unit 130 may reset the movement path according to the region of interest.

In relation to this, when the control unit 130 controls to rotate the moving body 101 the preset number of times, the control unit 130 may control to move the moving body 101 to the set region of interest.

To this end, the control unit 130 may control to rotate the moving body 101 towards the region of interest, by comparing the position information measured from the position measurement sensor with the position information set as the region of interest.

In this instance, when an object is detected from the object detection sensor while the moving body 101 is moving to the region of interest, the control unit 130 may control to stop the moving body 101, and the control unit 130 may control to carry out object detection again after the preset time interval.

In this instance, when the object is detected again after controlling to stop the moving body 101 due to the presence of the detected object, the control unit 130 may control to rotate the moving body 101 according to the preset steering angle, and the control unit 130 may control to move the moving body 101 by the preset threshold distance interval along the direction in which the moving body 101 has rotated.

Accordingly, the control unit 130 may control to rotate the moving body 101 towards the region of interest by comparing the position information measured at the location to which the moving body 101 has moved by the preset threshold distance interval with the position information set as the region of interest.

Meanwhile, when arriving at the location set as the region of interest, the control unit 130 may control to rotate the moving body 101 according to the preset steering angle.

The learning unit 140 may generate the path model to present the movable area for the moving body 101 by learning the position information changed with the movement of the moving body 101 along the movement path.

To this end, the learning unit 140 may use an unsupervised learning technique, for example, PCA, which analyzes input information and generates a pattern of the input information.

Accordingly, the control unit 130 may generate the movement path based on the generated path model, and the control unit 130 may control to move the moving body 101 along the generated movement path.

Additionally, when the region of interest is set, the control unit 130 may use the movable path according to the path model to move from another region to the region of interest.

In relation to this, the learning unit 140 may generate the path model according to the plurality of position information indicating the outermost side among the position information generated by the movement of the moving body 101.

Meanwhile, the control unit 130 may generate the movement path spaced the preset distance interval apart from the path model generated along the outer side of the collected position information. In this instance, generating the movement path may be understood as generating a path along which the mobile antiviral robot 100 moves according to the path model.

The control unit 130 may generate the straight line movement path spaced the predetermined distance apart from the previously generated movement path. In this case, the control unit 130 may generate the movement path in a grid shape within the movement path generated according to the position information of the outer side.

Additionally, the control unit 130 may set the movement path to rotate and move in the same direction at the branch point found in the generated movement path, and in this instance, when the previous movement path is found, the control unit 130 may set the movement path to rotate and move in a different direction.

Additionally, when the movement path of each direction found from the branch point is determined to be the previous movement path, the control unit 130 may set the movement path to move along the immediately previous movement path, and when arriving at the branch point where there is a movement path that has never been moved before, may set the movement path to rotate and move toward the corresponding movement path.

To this end, the control unit 130 may control to store the position information generated in a time series, and the control unit 130 may determine the previous movement path by comparing the position information generated for a period of time with the movement path.

Additionally, the control unit 130 may set the movement path to rotate in the same direction the preset number of times for the branch point found in the generated movement path, and in this case, when the number of rotations at the branch point reaches the preset number of times, the control unit 130 may set the movement path to rotate in a different direction.

Meanwhile, when the region of interest is set, the control unit 130 may use the movable path according to the path model to move from another region to the region of interest.

In relation to this, when arriving at a location more than the preset distance interval away from the region of interest, the control unit 130 may set to move from the corresponding location to the region of interest along the previously generated movement path.

In this instance, when the number of times it is beyond the circle according to the preset distance interval reaches the preset number of times, the control unit 130 may increase the preset distance interval by the predetermined amount.

Meanwhile, the control unit 130 may control to move along one movement path except the movement path that has been used before among at least one possible movement path to the region of interest.

To this end, the control unit 130 may recognize directions of different possible movement paths from the region of interest, and accordingly, the control unit 130 may select the directions of the movement paths recognized from the region of interest in a sequential order, and generate the movement path to move in different directions.

Additionally, the control unit 130 may recognize the branch point found along the movement path set from the region of interest, and accordingly, the control unit 130 may select the direction of the movement path including the branch point recognized along the movement path set from the region of interest in the process of selecting the direction of the movement path recognized from the region of interest in a sequential order.

Here, the control unit 130 may recognize the n-th branch point among the branch points found in a sequential order along the movement path set from the region of interest.

Additionally, when the region of interest is set to move from a location to the region of interest, the control unit 130 may select the direction of the movement path with the smallest number of rotations and set to move the mobile antiviral robot 100 to the region of interest.

Additionally, the control unit 130 may set, as the target point, any one of the branch points according to the generated movement path, and when arriving at the branch point set as the target point, the control unit 130 may set to move to the region of interest.

In this instance, the control unit 130 may set the branch point included in the previously set movement path as the passage point, and in this case, the control unit 130 may set, as the target point, any one branch point except the branch point set as the passage point.

Additionally, the control unit 130 may set the branch point present in the generated movement path as the target point in an ascending order of the distance from the region of interest, and the control unit 130 may set the branch point present in the generated movement path as the target point in a descending order of the distance from the region of interest.

The storage unit 150 may store the path model generated by the learning unit 140, and to this end, the storage unit 150 may store the position information measured by the sensor unit 120.

Additionally, the storage unit 150 may store a control command required for the control over the moving body 101 or the antiviral unit 110.

Accordingly, the storage unit 150 may store the control process performed by the control unit 130 and the control result, and the storage unit 150 may store the information measured by the sensor unit 120.

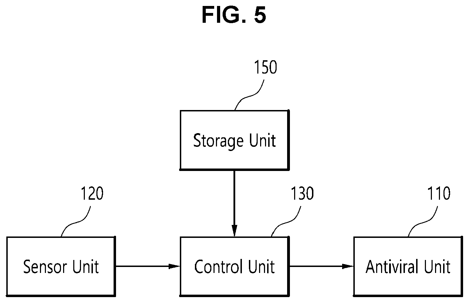

FIG. 5 is a block diagram showing a process of controlling the antivirus unit by the control unit of FIG. 4.

Referring to FIG. 5, the sensor unit 120 may include the pollution degree measurement sensor to measure the pollution degree of air entering the internal space by the rotation of the blower fan.

Accordingly, the control unit 130 may control the rotation speed of the blower fan according to the pollution degree measured by the pollution degree measurement sensor.

To this end, the control unit 130 may determine the pollution level according to the pollution degree measured by the pollution degree measurement sensor, and the control unit 130 may control the rotation speed of the blower fan according to the determined pollution level.

Here, the pollution level may be set such that the pollution degree is classified into at least one level according to the numerical range indicated by the pollution degree.

In this instance, when different styles of pollution degree measurement sensors are provided, each may be provided to determine the pollution level according to the pollution degree measured by each pollution degree measurement sensor, and the control unit 130 may control the rotation speed of the blower fan based on the plurality of pollution levels determined according to the pollution degree measured by the different styles of pollution degree measurement sensors.

In relation to this, the storage unit 150 may store a control command required for the control over the antiviral unit 110, and additionally, the storage unit 150 may store the control process performed by the control unit 130 and the control result, and the storage unit 150 may store the information measured by the sensor unit 120.

Meanwhile, the rotation speed of the blower fan may be controlled by the control unit 130.

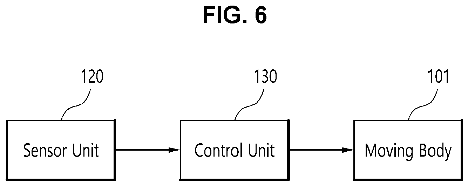

FIG. 6 is a block diagram showing a process of controlling to move the moving body by the control unit of FIG. 4.

Referring to FIG. 6, the sensor unit 120 may include the object detection sensor to transmit a detection signal and receive the detection signal reflected by an object to detect the object.

In this instance, the object detection sensor may be provided to transmit the detection signal towards the movement direction of the moving body 101, and accordingly, the sensor unit 120 may detect an object located in front of the sensor unit 120.

Accordingly, when the object is detected by the object detection sensor, the control unit 130 may set the movement path according to the status of the detected object, and the control unit 130 may control to move the moving body along the set movement path.

In this instance, when the object is detected, the control unit 130 may control to stop the moving body 101, and the control unit 130 may control to carry out object detection again after the preset time interval.

Additionally, when the object is detected again after controlling to stop the moving body 101 due to the presence of the detected object, the control unit 130 may control to rotate the moving body 101 according to the preset steering angle.

In relation to this, when the control unit 130 controls to rotate the moving body 101 according to the preset steering angle, the control unit 130 may control to rotate the moving body 101 in the same direction as the previous rotation direction.

Accordingly, the control unit 130 may carry out object detection towards the changed direction by the rotation of the moving body 101, and when the object is not detected from the changed direction, the control unit 130 may control to move the moving body 101 towards the changed direction.

Meanwhile, the control unit 130 may carry out object detection towards the changed direction by the rotation of the moving body 101, and when the object is detected, may control to carry out object detection again after the preset time interval, and when the object is detected again, the control unit 130 may control to rotate the moving body 101 according to the preset steering angle.

In relation to this, when it is determined that the movement distance of the moving body 101 from the location at which the first object is detected from the object detection sensor and the moving body 101 rotates according to the preset first steering angle to the location at which the second object is detected is within the preset distance range, the control unit 130 may control to rotate the moving body 101 according to the preset second steering angle.

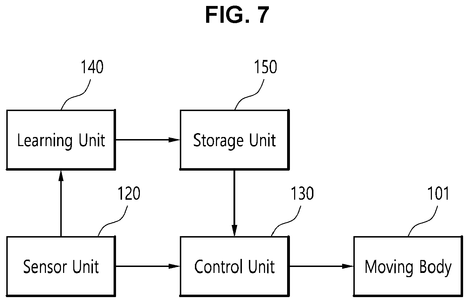

FIG. 7 is a block diagram showing a process of generating the path model by the control unit of FIG. 4.

Referring to FIG. 7, the sensor unit 120 may include the position measurement sensor to measure the position of the moving body to generate position information.

Accordingly, the learning unit 140 may generate the path model to present the movable area for the moving body 101 by learning the position information changed with the movement of the moving body 101 along the movement path.

To this end, the learning unit 140 may use an unsupervised learning technique, for example, PCA, which analyzes input information and generates a pattern of the input information.

In relation to this, the storage unit 150 may store the path model generated by the learning unit 140, and to this end, the storage unit 150 may store the position information measured by the sensor unit 120.

Accordingly, the control unit 130 may control to move the moving body 101 along the movement path set based on the generated path model.

Additionally, when the region of interest is set, the control unit 130 may use the movable path according to the path model to move from another region to the region of interest.

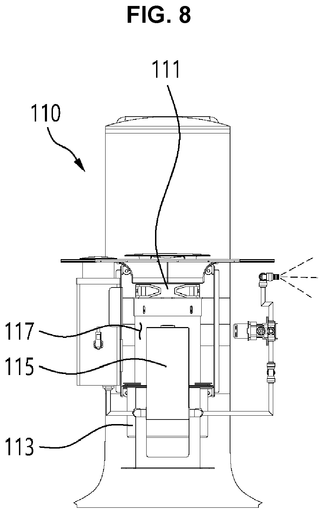

FIGS. 8 to 11 are schematic diagrams showing an embodiment of the antivirus unit of FIG. 4.

The antiviral unit 110 may further include the sprayer to spray the disinfectant solution onto the article such as the table and the chair.

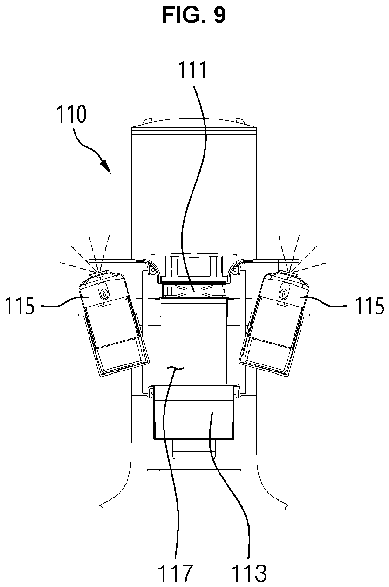

In relation to this, referring to FIG. 8, the mobile antiviral robot 100 may be understood as including the sprayer 115 on the front side of the antiviral unit 110, and referring to FIG. 9, the mobile antiviral robot 100 may be understood as including the sprayer 115 on two sides of the antiviral unit 110.

In this instance, the sprayer 115 may be provided outside of the internal space, and the cover 105 may include the hole through which the disinfectant solution sprayed from the sprayer 115 is delivered out of the mobile antiviral robot 100. In this instance, the sprayer 115 may further include the nozzle to spray the disinfectant solution out of the mobile antiviral robot 100, and in this case, the cover 105 may include the hole in which the nozzle of the sprayer 115 is positioned.

Additionally, when the mobile antiviral robot 100 includes the sprayer 115 to spray the disinfectant solution onto the article such as the table and the chair, the mobile antiviral robot 100 may spray the disinfectant solution onto the article such as the table and chair determined to be empty.

When the mobile antiviral robot 100 is positioned in the store, for example, a cafe, this may be understood as spraying the disinfectant solution onto the table and chair determined to be empty, and in this instance, the mobile antiviral robot 100 may spray the disinfectant solution in response to the spray signal of the disinfectant solution received from the seat control system connected with the wireless or wired network.

The sprayer 115 may be provided to spray the disinfectant solution in response to the external input spray signal, and here, the spray signal may be a signal using the wireless network such as Wi-Fi and Bluetooth, and the spray signal may be generated by a device which can connect to the wireless network, for example, a computer, a smartphone and a tablet.

In this instance, the mobile antiviral robot 100 may be connected to the device which generates the spray signal with the wireless network.

As described above, the sprayer 115 may be implemented in other embodiment without departing from the spirit and scope of the present disclosure in relation to an embodiment of the particular shape, structure and feature as described herein.

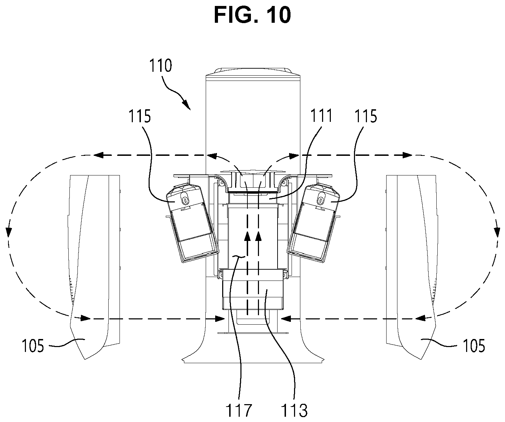

Meanwhile, referring to FIG. 10, the direction of movement of air by the blower fan 111 provided on the upper side of the antiviral unit 110 can be briefly seen through a dashed line.

To this end, the antiviral unit 110 may include the rotatable blower fan 111 to allow air to enter the internal space 117 formed by the at least one cover 105, and the antiviral unit 110 may include the purification filter 113 to purify air entering the internal space 117 by the blower fan 111.

The blower fan 111 may be provided on the upper side of the internal space 117 formed by the cover 105, and may rotate to force air entering the internal space 117 out of the internal space 117.

In this case, the antiviral unit 110 may be controlled to allow air to enter the lower side or the side of the internal space 117 by the blower fan 111, and the antiviral unit 110 may be controlled to allow the incoming air to pass through the blower fan 111 and exit through the upper side of the internal space 117.

Additionally, the blower fan 111 may be provided on the lower side of the internal space 117 formed by the cover 105, and may rotate to move air inside the internal space 117 to the upper side of the internal space 117.

In this case, the antiviral unit 110 may be controlled to move air inside the internal space 117 to the upper side of the internal space 117 by the blower fan 111, and force the air having moved to the upper side of the internal space 117 out of the internal space 117, and accordingly, the antiviral unit 110 may let air into the internal space 117 from the lower side or the side of the internal space 117.

In this instance, the blower fan 111 may be provided such that its rotation speed is controlled by the control unit 130.

Meanwhile, the purification filter 113 may be installed in the internal space 117 formed by the cover 105. In this instance, the purification filter 113 may be provided to capture or filter out pollutants in air.

In relation to this, the purification filter 113 may include a plurality of single filters in different shapes, and here, the plurality of single filters may be provided as filters for capturing or filtering out different pollutants.

For example, the single filter may include a pre filter for capturing dust with large particles in air, an activated carbon filter for capturing odor, hazardous gas or particles of TVOC in air, and a H13 HEPA Filter for capturing particles in air, for example, ultrafine dust of 2.5 micrometers or less, and in this case, the purification filter may be provided in the form of a triple filter including the pre filter, the activated carbon filter and the H13 HEPA filter.

The single filter may be of a mesh type to allow air to pass through and filter out pollutants, and in this instance, the single filter may be made of metal or synthetic resin.

Additionally, the purification filter may include the plurality of different single filters provided for different purposes according to the feature of the space in which the mobile antiviral robot 100 works, and it is obvious that the purification filter may include a plurality of different well-known filters.

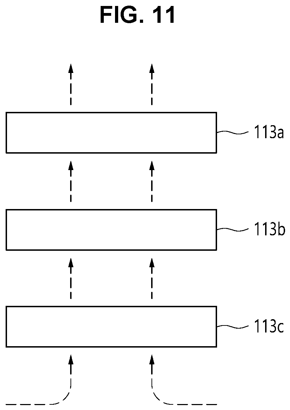

In relation to this, referring to FIG. 11, the plurality of different single filters 113a, 113b, 113c are shown, and the antiviral unit 110 may be provided to allow air moving by the rotation of the blower fan 111 to pass through the plurality of different single filters 113a, 113b, 113c.

Meanwhile, the purification filter 113 may be provided to be secured to the inner side of the cover 105 that forms the internal space 117, and additionally, the purification filter 113 may be secured around the internal space 117 formed by the cover 105. Additionally, the purification filter 113 may be provided in the internal space 117 so that air entering from the lower side or the side of the internal space 117 passes through the purification filter 113 by the operation of the blower fan 111.

In this case, the internal space 117 formed by the cover 105 may be provided with an assembly in the shape of a tube with a hollow center to which the purification filter 113 is secured, and accordingly, the antiviral unit 110 may control the blower fan 111 to move air purified by the purification filter 113 into the assembly in the shape of a tube with a hollow center, and force the purified air inside the assembly out of the internal space 117 formed by the cover 105.

In this instance, the purification filter 113 may be replaceably provided.

Additionally, the mobile antiviral robot 100 may further include the sprayer 115 to spray the disinfectant solution onto the article such as the table and the chair.

In this instance, the sprayer 115 may be provided outside of the internal space, and the cover 105 may include the hole through which the disinfectant solution sprayed from the sprayer 115 is delivered out of the mobile antiviral robot 100. In this instance, the sprayer 115 may further include the nozzle to spray the disinfectant solution out of the mobile antiviral robot 100, and in this case, the cover 105 may include the hole in which the nozzle of the sprayer 115 is positioned.

Additionally, when the mobile antiviral robot 100 includes the sprayer 115 to spray the disinfectant solution onto the article such as the table and the chair, the mobile antiviral robot 100 may spray the disinfectant solution onto the article such as the table and chair determined to be empty.

When the mobile antiviral robot 100 is positioned in the store, for example, a cafe, this may be understood as spraying the disinfectant solution onto the table and the chair determined to be empty, and in this instance, the mobile antiviral robot 100 may spray the disinfectant solution in response to the spray signal of the disinfectant solution received from the seat control system connected with the wireless or wired network.

Meanwhile, the mobile antiviral robot 100 may include the disinfection lamp provided on the lower surface of the moving body 101 to emit UV radiation towards the floor on which the moving body 101 moves.

In this case, the mobile antiviral robot 100 may disinfect the floor using the disinfection lamp when the moving body 101 stands by or while the moving body 101 is moving.

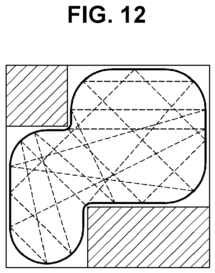

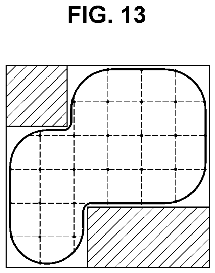

FIGS. 12 and 13 are schematic diagrams showing an embodiment of the movement path generated by the control unit of FIG. 4.

In relation to this, the learning unit 140 may generate the path model to present the movable area for the moving body 101 by learning the position information changed with the movement of the moving body 101 along the movement path.

Accordingly, the control unit 130 may generate the movement path based on the generated path model, and the control unit 130 may control to move the moving body 101 along the generated movement path.

Additionally, when the region of interest is set, the control unit 130 may use the movable path according to the path model to move from another region to the region of interest.

In relation to this, the learning unit 140 may generate the path model according to the plurality of position information indicating the outermost side among the position information generated by the movement of the moving body 101.

Meanwhile, the control unit 130 may generate the movement path spaced the preset distance interval apart from the path model generated along the outer side of the collected position information. In this instance, generating the movement path may be understood as generating a path along which the mobile antiviral robot 100 moves according to the path model.

The control unit 130 may generate the straight line movement path spaced the predetermined distance apart from the previously generated movement path. In this case, the control unit 130 may generate the movement path in a grid shape within the movement path generated according to the position information of the outer side.

Additionally, the control unit 130 may set the movement path to rotate and move in the same direction at the branch point found in the generated movement path, and in this instance, when the previous movement path is found, the control unit 130 may set the movement path to rotate and move in a different direction.

Additionally, when the movement path of each direction found from the branch point is determined to be the previous movement path, the control unit 130 may set the movement path to move along the immediately previous movement path, and when arriving at the branch point where there is a movement path that has never been moved before, may set the movement path to rotate and move toward the corresponding movement path.

To this end, the control unit 130 may control to store the position information generated in a time series, and the control unit 130 may determine the previous movement path by comparing the position information generated for a period of time with the movement path.

Additionally, the control unit 130 may set the movement path to rotate in the same direction the present number of times for the branch point found in the generated movement path, and in this case, when the number of rotations at the branch point reaches the preset number of times, the control unit 130 may set the movement path to rotate in a different direction.

Meanwhile, when the region of interest is set, the control unit 130 may use the movable path according to the path model to move from another region to the region of interest.

In relation to this, when arriving at a location more than the preset distance interval away from the region of interest, the control unit 130 may set to move from the corresponding location to the region of interest along the previously generated movement path.

In this instance, when the number of times it is beyond the circle according to the preset distance interval reaches the preset number of times, the control unit 130 may increase the preset distance interval by the predetermined amount.

Meanwhile, the control unit 130 may control to move along one movement path except the movement path that has been used before among at least one possible movement path to the region of interest.

To this end, the control unit 130 may recognize directions of different possible movement paths from the region of interest, and accordingly, the control unit 130 may select the directions of the movement paths recognized from the region of interest in a sequential order, and generate the movement path to move in different directions.

Additionally, the control unit 130 may recognize the branch point found along the movement path set from the region of interest, and accordingly, the control unit 130 may select the direction of the movement path including the branch point recognized along the movement path set from the region of interest in the process of selecting the direction of the movement path recognized from the region of interest in a sequential order.

Here, the control unit 130 may recognize the n-th branch point among the branch points found in a sequential order along the movement path set from the region of interest.

Additionally, when the region of interest is set to move from a location to the region of interest, the control unit 130 may select the direction of the movement path with the smallest number of rotations and set the mobile antiviral robot 100 to move to the region of interest.

Additionally, the control unit 130 may set any one of the branch points on the generated movement path as the target point, and when arriving at the branch point set as the target point, the control unit 130 may set to move to the region of interest.

In this instance, the control unit 130 may set the branch point included in the previously set movement path as the passage point, and in this case, the control unit 130 may set, as the target point, any one branch point except the branch point set as the passage point.

Additionally, the control unit 130 may set the branch point present in the generated movement path as the target point in an ascending order of the distance from the region of interest, and the control unit 130 may set, as the target point, the branch point present in the generated movement path in a descending order of the distance from the region of interest.

The storage unit 150 may store the path model generated by the learning unit 140, and to this end, the storage unit 150 may store the position information measured by the sensor unit 120.

Additionally, the storage unit 150 may store a control command required for the control over the moving body 101 or the antiviral unit 110.

Accordingly, the storage unit 150 may store the control process performed by the control unit 130 and the control result, and the storage unit 150 may store the information measured by the sensor unit 120.

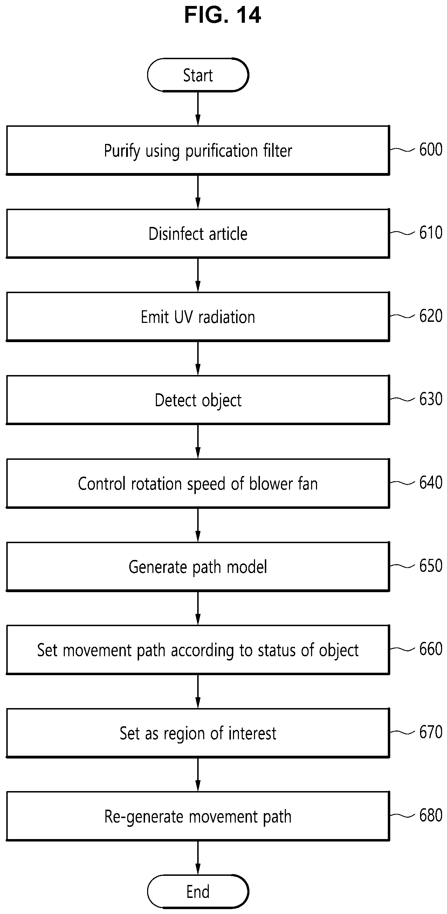

FIG. 14 is a flowchart of a method for controlling a mobile antiviral robot according to an embodiment of the present disclosure.

The method for controlling a mobile antiviral robot according to an embodiment of the present disclosure is performed on substantially the same configuration as the mobile antiviral robot 100 shown in FIG. 1, and the same reference sign is given to the same element as the mobile antiviral robot 100 of FIG. 1, and redundant description is omitted.

The method for controlling a mobile antiviral robot may include purifying using the purification filter (600), disinfecting an article (610), emitting UV radiation (620), detecting an object (630), controlling the rotation speed of the blower fan (640), generating a path model (650), setting a movement path according to the status of the object (660), setting as a region of interest (670) and re-generating the movement path (680).

The step 600 of purifying using the purification filter may be a step of purifying, using the purification filter, air moving by the blower fan which rotates to induce the movement of the air.

The step 610 of disinfecting the article may be a step of spraying the disinfectant solution to disinfect the article.

The step 620 of emitting UV radiation may be a step of emitting UV radiation from the lower surface of the moving body towards the floor.

The step 630 of detecting the object may be a step of measuring the pollution degree of air, measuring the position of the moving body to generate position information, and detecting an object located in the movement direction of the moving body.

The step 640 of controlling the rotation speed of the blower fan may be a step of controlling the rotation speed of the blower fan according to the pollution degree.

The step 650 of generating the path model may be a step of generating the path model to present the movable area for the moving body by learning the position information changed with the movement of the moving body.

The step 660 of setting the movement path according to the status of the object may be a step of, when the object is detected, setting the movement path according to the status of the detected object.

The step 670 of setting the region of interest may be a step of, when the pollution degree is outside of the preset pollution threshold range, setting the position information as the region of interest.

The step 680 of re-generating the movement path may be a step of generating the movement path spaced the preset distance interval apart from the path model, and re-generating the straight line movement path spaced the predetermined distance apart from the previously generated movement path.

While the present disclosure has been hereinabove described with reference to the embodiments, those skilled in the art will understand that a variety of modifications and changes may be made thereto without departing from the spirit and scope of the present disclosure defined in the appended claims.

### [Detailed Description of Main Elements]

100: Mobile antiviral robot
101: Moving body
103: Body
105: Cover
111: Blower fan
113: Purification filter
115: Sprayer
117: Internal space

## Claims

1. A mobile antiviral robot including a moving body to allow the mobile antiviral robot to move, the mobile antiviral robot comprising:
a first antiviral unit including a blower fan to induce movement of air and a purification filter to purify the air moving by the blower fan;
a second antiviral unit provided to spray a disinfectant solution to disinfect an article;
a third antiviral unit provided on a lower surface of the moving body to emit ultraviolet radiation towards a floor;
a sensor unit to measure a pollution degree of the air, detect an object located in a movement direction of the moving body, and measure a position of the moving body to generate position information;
a learning unit to generate a path model to present a movable area for the moving body by learning the position information changed with the movement of the moving body; and
a control unit to control a rotation speed of the blower fan according to the pollution degree, when the object is detected, set a movement path according to a status of the detected object, when the pollution degree is outside of a preset pollution threshold range, set the position information as a region of interest, generate a movement path spaced a preset distance interval apart from the path model, and re-generate a straight line movement path spaced the predetermined distance apart from the previously generated movement path.

2. The mobile antiviral robot according to claim 1, wherein the control unit sets the movement path to rotate in a same direction a preset number of times for a branch point found in the generated movement path, and when a number of rotations at the branch point reaches the preset number of times, sets the movement path to rotate the moving body in a different direction.

3. The mobile antiviral robot according to claim 1, wherein the control unit controls the second antiviral unit to spray the disinfectant solution when the control unit receives a spray signal according to seat information of a location adjacent to the moving body from a seat control system provided to determine a seat status of a store indicating an empty seat and an occupied seat.

4. The mobile antiviral robot according to claim 1, wherein when the object is detected, the control unit controls to stop the moving body, and controls to detect the object again after a preset time interval, and when the object is detected again, controls to rotate the moving body according to a preset steering angle.

5. The mobile antiviral robot according to claim 4, wherein when it is determined that a movement distance of the moving body from a location at which a first object is detected and the moving body rotates according to a preset first steering angle to a location at which a second object is detected is within a preset distance range, the control unit controls to rotate the moving body according to a preset second steering angle.

6. The mobile antiviral robot according to claim 1, wherein when the moving body arrives at a location more than the preset distance interval away from the region of interest, the control unit sets to move the moving body from the corresponding location to the region of interest along the previously generated movement path.

7. The mobile antiviral robot according to claim 6, wherein when a number of arrivals at the location more than the preset distance interval away reaches a preset number of times, the control unit increases the preset distance interval by a predetermined amount.

8. The mobile antiviral robot according to claim 1, wherein the control unit sets any one of branch points along the generated movement path as a target point, and sets to move to the region of interest when arriving at the branch point set as the target point, wherein the branch point present in the movement path is set as the target point in a sequential order in an ascending order of distance from the region of interest.

9. A method for controlling a mobile antiviral robot including a moving body to allow the mobile antiviral robot to move, the method comprising:
purifying air using a purification filter, wherein the air moves by a blower fan which rotates to induce the movement of the air;
spraying a disinfectant solution to disinfect an article;
emitting ultraviolet radiation from a lower surface of the moving body towards a floor;
measuring a pollution degree of the air, measuring a position of the moving body to generate position information, and detecting an object located in a movement direction of the moving body;
controlling a rotation speed of the blower fan according to the pollution degree;
generating a path model to present a movable area for the moving body by learning the position information changed with the movement of the moving body;
setting a movement path according to a status of the detected object when the object is detected;
setting the position information as a region of interest when the pollution degree is outside of a preset pollution threshold range; and
generating a movement path spaced a preset distance interval apart from the path model, and re-generating a straight line movement path spaced the predetermined distance apart from the previously generated movement path.

10. The method for controlling a mobile antiviral robot according to claim 9, wherein re-generating the movement path comprises setting any one of branch points along the generated movement path as a target point, and setting to move to the region of interest when arriving at the branch point set as the target point, wherein the branch point present in the movement path is set as the target point in a sequential order in an ascending order of distance from the region of interest.

11. The method for controlling a mobile antiviral robot according to claim 9, wherein spraying the disinfectant solution to disinfect the article is performed when receiving a spray signal according to seat information of a location adjacent to the moving body from a seat control system provided to determine a seat status of a store indicating an empty seat and an occupied seat.

12. The method for controlling a mobile antiviral robot according to claim 11, wherein re-generating the movement path comprises setting the movement path to rotate in a same direction a preset number of times for the branch point found in the generated movement path, and when a number of rotations at the branch point reaches the preset number of times, setting the movement path to rotate the moving body in a different direction.
